# EUROPÄISCHE PATENTSCHRIFT

(11) **EP 3 764 868 B1**
(45) Veröffentlichungstag und Bekanntmachung des Hinweises auf die Patenterteilung: **22.02.2023**
(21) Anmeldenummer: 19709675.3
(22) Anmeldetag: 05.03.2019
(51) Int. Cl.: A61B 1/00, G02B 23/24

(54) **OPTISCHES SYSTEM FÜR EIN ENDOSKOP**
OPTICAL SYSTEM FOR AN ENDOSCOPE
SYSTÈME OPTIQUE POUR ENDOSCOPE

(30) Priorität: 14.03.2018 DE 102018105846
(43) Veröffentlichungstag der Anmeldung: 20.01.2021
(73) Patentinhaber: OLYMPUS Winter & Ibe GmbH, 22045 Hamburg (DE)
(72) Erfinder: WIETERS, Martin, 22885 Barsbüttel (DE); SCHÖLER, Uwe, 22955 Hoisdorf (DE)
(74) Vertreter: Seemann & Partner Patentanwälte mbB
(86) Internationale Anmeldenummer: PCT/EP2019/055406
(87) Internationale Veröffentlichungsnummer: WO 2019/174967

(56) Entgegenhaltungen:
- WO-A2-02/087426
- DE-A1-102007 032 202
- US-A- 5 860 912
- US-A1- 2006 058 581
- US-B1- 6 767 322

## Beschreibung

Die Erfindung betrifft ein optisches System für ein Endoskop, umfassend eine distale optische Baugruppe und eine proximale optische Baugruppe. Weiterhin betrifft die Erfindung ein Endoskop mit einem optischen System.

Bei medizinischen Endoskopen ist ein geringer Außendurchmesser des Endoskopschafts wünschenswert. Durch die zunehmende Miniaturisierung stellt die Herstellung und Montage des optischen Systems eine technische Herausforderung dar. So muss beispielsweise bei der Miniaturisierung des optischen Systems gewährleistet sein, dass die optischen Eigenschaften des Endoskops den Vorgaben entsprechen und das Endoskop gleichzeitig den Belastungen standhält, denen es im Betrieb und bei der Aufbereitung ausgesetzt wird. Dies gilt vor allem auch für die Halterungen im optischen System, die Komponenten wie beispielsweise optische Elemente und Bildsensoren aufnehmen.

Stereo-Videoendoskope verfügen über zwei voneinander getrennte Linsensystemkanäle, die jeweils Lichtbündel aus einem Blickfeld mit leicht unterschiedlichem Blickwinkel auf jeweils einem Bildsensor abbilden. Auf diese Weise lässt sich ein Stereobild des Aufnahmebereichs zusammensetzen, das einem Betrachter einen räumlichen Eindruck vermittelt. Die Verwendung von zwei Linsensystemkanälen erfordert eine besonders platzsparende Ausführung des optischen Systems, um den Außendurchmesser des Stereo-Videoendoskops nicht unnötig zu vergrößern.

WO 02/087426 A2 offenbart ein Endoskop, welches an seinem distalen Ende einen Wechselkopf umfasst. Da der Kopf des Endoskops auswechselbar ist, können auf das Endoskop unterschiedliche distale Optiken, in Form unterschiedlicher Wechselköpfe, aufgesetzt werden. Der Wechselkopf ist über zwei Positionierstifte, die in zugehörige Bohrungen eingeschoben werden, mit dem distalen Ende des Endoskops verbunden. In dem Wechselkopf befinden sich mehrere Linsen. In dem distalen Ende des Endoskopschafts ist ein Faserbündel vorhanden, welches der Bildübertragung durch den Endoskopschaft dient.

In DE 10 2007 032 202 A1 ist ein Endoskop gezeigt, dessen distales Ende und dessen proximaler Handgriff über einen Zapfen miteinander verbunden sind. In dem proximalen Handgriff befinden sich optische Elemente, beispielsweise Linsen. Bei dem Zapfen handelt es sich um ein Bündel von Faseroptiken.

Aus US 2006/0058581 A1 ist ein Schrägsichtvideoendoskop bekannt. Dieses umfasst eine Umlenkprismengruppe, welche hinter einer Eintrittslinse angeordnet ist. Weitere Linsen und ein Bildsensor sind gemeinsam mit diesen optischen Elementen in einem Hüllrohr angeordnet.

Ein Stereovideoendoskop ist in US 5,860,912 A gezeigt. Mithilfe eines linken und eines rechten Kanals erfolgt eine Abbildung auf einen linken und einen rechten Bildsensor. Die Bauteile werden in einem gemeinsamen Hüllrohr gehalten.

Ein weiteres Endoskop zeigt US 6,767,322 B1. Dieses umfasst ein distales Eintrittsfenster, hinter dem weitere Linsen angeordnet sind. Ferner ist ein Deckglas, ein Infrarotfilter und ein CCD-Chip umfasst. Das Deckglas ist von einem Halterahmen gehalten.

Es ist eine Aufgabe der Erfindung, ein optisches System für ein Endoskop und ein Endoskop anzugeben, wobei eine Halterung für das optische System einen stabilen und zuverlässigen Aufbau bei kleinem Bauraum aufweisen soll.

Die Aufgabe wird gelöst durch ein optisches System für ein Endoskop, umfassend eine distale optische Baugruppe und eine proximale optische Baugruppe, das dadurch weitergebildet ist, dass das optische System wenigstens ein hohlraumfreies stabförmiges Fixierelement mit einer längserstreckten Form aufweist, wobei mittels des stabförmigen Fixierelements die proximale optische Baugruppe an der distalen optischen Baugruppe befestigt ist und die proximale optische Baugruppe eine erste Halterung und eine zweite Halterung umfasst, die jeweils zur Aufnahme eines Bildsensors eingerichtet sind, wobei die erste Halterung gegenüber der zweiten Halterung achsensymmetrisch in Bezug auf eine Längsachse des optischen Systems angeordnet ist, und wobei ferner die erste Halterung und die zweite Halterung aneinander fixiert sind.

Vorzugsweise erstreckt sich das stabförmige Fixierelement in eine Richtung parallel zu einer Längsachse des optischen Systems. Durch die Ausrichtung des Fixierelements in eine Richtung parallel zur Längsachse des optischen Systems wird die Ausrichtung der proximalen optischen Baugruppe in Bezug auf die distale optische Baugruppe vereinfacht. Auch eine Ausrichtung schräg zur Längsachse des optischen Systems kann vorgesehen sein.

Im Kontext der vorliegenden Beschreibung wird unter einer distalen optischen Baugruppe eine optische Baugruppe verstanden, die distal der ebenfalls erwähnten proximalen optischen Baugruppe liegt. Gleiches gilt für die proximale optische Baugruppe.

Die distale optische Baugruppe umfasst insbesondere eine Eintrittslinse. Die proximale optische Baugruppe umfasst insbesondere wenigstens einen Bildsensor.

Vorteilhaft wird durch ein Fixierelement mit einer längserstreckten Form eine raumsparende Fixierung erreicht. Unter einer längserstreckten Form des Fixierelements wird ein Fixierelement verstanden, welches beispielsweise die Form eines länglichen Stifts oder Stabs aufweist. In jedem Fall ist das Fixierelement länglich, d.h. es weist eine Längserstreckungsrichtung auf, in der seine Dimension größer oder wesentlich größer ist, als in eine Richtung senkrecht zu dieser Längserstreckungsrichtung. Beispielsweise ist eine Länge des Fixierelements zumindest doppelt so groß wie sein Durchmesser.

Anders als bei Varianten zur Fixierung, bei denen eine der Baugruppen die andere Baugruppe ringförmig umschließt, ist bei einer Fixierung mit einem oder mehreren Fixierelement(en) eine Vergrö-ßerung der umschließenden Baugruppe in radialer Richtung überflüssig.

Bevorzugt weist das stabförmige Fixierelement eine zylinderförmige Form auf, wobei sich eine Zylinderachse des stabförmigen Fixierelements parallel zur Längsachse des optischen Systems erstreckt. Ein zylinderförmiges Fixierelement ist einerseits leicht herzustellen, andererseits sind die Mantelflächen und Deckflächen eines Zylinders gut als Kontaktflächen für eine Fixierung geeignet.

Das Fixierelement ist stabförmig. Das Fixierelement ist also ein Vollzylinder und kein Hohlzylinder, in seinem Innenraum sind keine optischen Elemente angeordnet. Insbesondere weist das Fixierelement einen kreisförmigen Querschnitt auf.

Das Fixierelement ist bevorzugt ein starrer, nicht flexibler oder biegbarer Körper. Das Fixierelement ist hohlraumfrei. Die starre, hohlraumfreie Ausbildung des Fixierelements stellt eine besonders stabile Ausgestaltung dar.

Der von dem Fixierelement eingenommene Raum liegt insbesondere vollständig außerhalb des Strahlengangs des in das optische System einfallenden Lichts.

Das Fixierelement verbindet insbesondere eine Fassung der distalen optischen Baugruppe, in der wenigstens ein optisches Element aufgenommen ist, mit einer Halterung der proximalen optischen Baugruppe, in der wenigstens ein weiteres optisches Element und/oder ein Bildsensor aufgenommen sind/ist.

Insbesondere steht das stabförmige Fixierelement in proximaler Richtung aus einer Stirnseite der Fassung der distalen optischen Baugruppe hervor. Die Stirnseite der Fassung schließt die distale optische Baugruppe in proximaler Richtung ab. Ein Durchmesser des stabförmigen Fixierelements ist insbesondere kleiner als ein Durchmesser der Fassung. Weiterhin ist der Durchmesser des stabförmigen Fixierelements insbesondere kleiner als ein Durchmesser des wenigstens einen in der Fassung aufgenommenen optischen Elements.

Gemäß einer Ausführungsform ist das stabförmige Fixierelement einstückig mit der distalen optischen Baugruppe hergestellt. In dieser Ausführungsform ist das stabförmige Fixierelement also beispielsweise als Teil der Fassung der distalen optischen Baugruppe ausgebildet. Es wird gemeinsam mit dieser Fassung einstückig als Teil der Fassung hergestellt.

Gemäß einer alternativen Ausführungsform ist das stabförmige Fixierelement als separates Element hergestellt und in einer Fassung der distalen optischen Baugruppe fixiert, insbesondere eingeschweißt. In dieser Ausführungsform werden die distale optische Baugruppe und das stabförmige Fixierelement separat hergestellt. Dies hat den Vorteil, dass sowohl die Fertigung der distalen optischen Baugruppe oder der Fassung der distalen optischen Baugruppe als auch die Herstellung des stabförmigen Fixierelements vereinfacht ist. Durch Einschweißen des stabförmigen Fixierelements in die Fassung der distalen Baugruppe wird eine stabile Fixierung erreicht. Es ist ebenso vorgesehen, das stabförmige Fixierelement einzulöten oder einzukleben.

Bevorzugt ist das stabförmige Fixierelement aus einem Grundmaterial hergestellt und mit einer Beschichtung versehen, wobei die Beschichtung eine bessere Lötbarkeit als das Grundmaterial aufweist.

Weiterhin bevorzugt ist das stabförmige Fixierelement aus dem Grundmaterial und die Fassung der distalen optischen Baugruppe aus einem Fassungsmaterial hergestellt, wobei sich das Grundmaterial von dem Fassungsmaterial unterscheidet.

Die Auswahl des Fassungsmaterials ist durch die angestrebten Eigenschaften der distalen optischen Baugruppe begrenzt. Vorteilhaft ist es aber durch die Herstellung des stabförmigen Fixierelements als separates Element möglich, als Grundmaterial des Fixierelements ein anderes Material als das Fassungsmaterial zu wählen. Durch die Wahl des Grundmaterials kann beispielsweise die Wärmeleitfähigkeit und/oder der Wärmeausdehnungskoeffizient des stabförmigen Fixierelements angepasst werden. Als Beschichtung des stabförmigen Fixierelements kann ein Material gewählt werden, das für die Fixierung an der distalen optischen Baugruppe und/oder der proximalen optischen Baugruppe gut geeignet ist.

Das stabförmige Fixierelement ist bevorzugt mittels einer Lötverbindung an der proximalen optischen Baugruppe fixiert. Als Lötverfahren wird beispielsweise Laserlöten eingesetzt. Folglich ist es vorteilhaft, als Beschichtung des stabförmigen Fixierelements ein Material mit guter Lötbarkeit wie beispielsweise Gold, Silber oder Zinn zu wählen.

Erfindungsgemäß umfasst die proximale optische Baugruppe eine erste Halterung und eine zweite Halterung, die jeweils zur Aufnahme eines Bildsensors eingerichtet sind, wobei die erste Halterung gegenüber der zweiten Halterung achsensymmetrisch im Bezug zur Längsachse des optischen Systems ist, und wobei die erste Halterung und die zweite Halterung aneinander fixiert sind.

Dieses optische System ist für ein Stereo-Videoendoskop besonders vorteilhaft. Die proximale optische Baugruppe umfasst die beiden Halterungen sowie die auf den Halterungen angeordneten Komponenten. Durch die symmetrische Anordnung wird eine raumsparende Bauweise der proximalen optischen Baugruppe erreicht. Indem die erste Halterung und die zweite Halterung aneinander fixiert sind, wird die Stabilität der proximalen optischen Baugruppe erhöht.

Bevorzugt ist, in radialer Richtung, ein Umfangskreis um beide Halterungen zusammen, im Wesentlichen gleich einem Umfangskreis um die Fassung der distalen optischen Baugruppe. Die radiale Richtung ist senkrecht zur Längsachse des optischen Systems, so dass die Fläche der Umfangskreise senkrecht zur Längsachse ist. Der Außendurchmesser der distalen optischen Baugruppe entspricht also im Wesentlichen dem Außendurchmesser der distalen optischen Baugruppe. Vorteilhaft wird auf diese Weise ein raumsparender Aufbau des optischen Systems realisiert.

In einer Ausführungsform umfasst das optische System ein erstes stabförmiges Fixierelement und ein zweites stabförmiges Fixierelement, wobei das erste stabförmige Fixierelement die erste Halterung und das zweite stabförmige Fixierelement die zweite Halterung an der distalen optischen Baugruppe fixiert. Vorteilhaft ist für jede Halterung jeweils ein stabförmiges Fixierelement vorgesehen, mit dem diese Halterung mit der distalen optischen Baugruppe fixiert wird.

Gemäß einer alternativen Ausführungsform umfasst das optische System ein erstes stabförmiges Fixierelement und ein zweites stabförmiges Fixierelement, wobei jede Halterung jeweils einen ersten Fixierbereich und einen zweiten Fixierbereich aufweist, wobei das erste stabförmige Fixierelement die distale optische Baugruppe an dem ersten Fixierbereich der zweiten Halterung und dem zweiten Fixierbereich der ersten Halterung fixiert, und wobei das zweite stabförmige Fixierelement die distale optische Baugruppe an dem ersten Fixierbereich der ersten Halterung und dem zweiten Fixierbereich der zweiten Halterung fixiert. Vorteilhaft umfasst in dieser Ausführungsform jede Halterung zwei Fixierbereiche, die jeweils an einem der beiden stabförmigen Fixierelemente befestigt sind. Mit anderen Worten ist also jede der beiden Halterungen an jedem der beiden stabförmigen Fixierelemente befestigt. Diese Befestigung "über Kreuz" sorgt für einen besonders stabilen Aufbau, d.h. es wird eine besonders stabile Fixierung der Halterungen und der proximalen optischen Baugruppe an der distalen optischen Baugruppe realisiert.

Bevorzugt umfasst das optische System einen ersten Linsensystemkanal mit einer ersten optischen Achse und einen zweiten Linsensystemkanal mit einer zweiten optischen Achse, wobei die erste optische Achse parallel zur zweiten optischen Achse und zur Längsachse des optischen Systems verläuft, wobei ein erstes stabförmiges Fixierelement oberhalb des ersten Linsensystemkanals und ein zweites stabförmiges Fixierelement unterhalb des zweiten Linsensystemkanals angeordnet sind. Die Begriffe oberhalb und unterhalb sind dabei in Bezug auf eine Ebene bezogen, die durch die optischen Achsen aufgespannt wird. Durch diese Anordnung der stabförmigen Fixierelemente wird die Stabilität der Fixierung der an den Fixierelementen befestigten proximalen optischen Baugruppe oder Halterungen erhöht. Insbesondere sind zwei Halterungen über Kreuz an den stabförmigen Fixierelementen befestigt, so dass jede Halterung an beiden Fixierelementen fixiert ist.

Das optische System ist also bevorzugt ein optisches System eines Stereo-Videoendoskops. Die Begriffe der distalen optischen Baugruppe und der proximalen optischen Baugruppe werden im Kontext der vorliegenden Beschreibung und im Kontext eines Stereo-Videoendoskops so verstanden, dass sowohl die distale als auch die proximale optische Baugruppe ein Teil einer Baugruppe sind, in der die beiden optischen Kanäle (linker Kanal und rechter Kanal) separat geführt werden.

Die Aufgabe wird außerdem gelöst durch ein Endoskop, insbesondere Stereo-Videoendoskop, umfassend ein optisches System gemäß einer der zuvor beschriebenen Ausführungsformen.

Auch auf das Endoskop treffen gleiche oder ähnliche Vorteile zu, wie sie bereits oben im Hinblick auf das optische System erwähnt wurden, so dass auf Wiederholungen verzichtet werden soll. Weitere Merkmale der Erfindung werden aus der Beschreibung erfindungsgemäßer Ausführungsformen zusammen mit den Ansprüchen und den beigefügten Zeichnungen ersichtlich.

Die Erfindung wird nachstehend anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine abschnittsweise schematisch vereinfachte Darstellung eines Endoskops,
- Fig. 2: eine schematisch vereinfachte perspektivische Darstellung eines optischen Systems für ein Stereo-Videoendoskop mit zwei Halterungen,
- Fig. 3: eine schematisch vereinfachte perspektivische Darstellung einer distalen Baugruppe eines optischen Systems für ein Stereo-Videoendoskop mit zwei Fixierelementen,
- Fig. 4: eine schematisch vereinfachte perspektivische Darstellung eines optischen Systems für ein Stereo-Videoendoskop mit zwei Halterungen in einer gedrehten Ansicht,
- Fig. 5: eine schematisch vereinfachte perspektivische Darstellung einer Halterung für ein optisches System eines Endoskops und
- Fig. 6a bis 6d: eine schematisch vereinfachte Darstellung eines Verfahrens, das nicht unter den Gegenstand der Ansprüche fällt, zum Fixieren einer proximalen optischen Baugruppe an einer distalen optischen Baugruppe.

In den Zeichnungen sind jeweils gleiche oder gleichartige Elemente und/oder Teile mit denselben Bezugsziffern versehen, so dass von einer erneuten Vorstellung jeweils abgesehen wird.

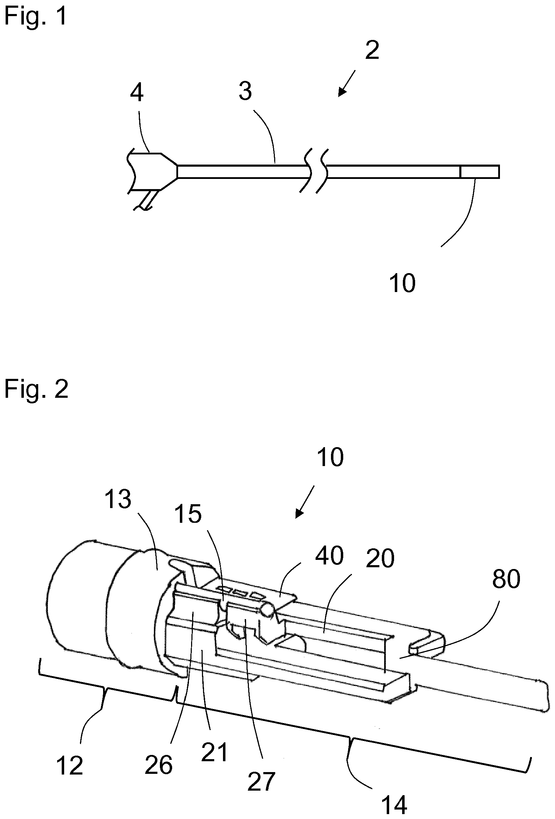

Fig. 1 zeigt abschnittsweise in schematisch vereinfachter Darstellung ein Endoskop 2 mit einem Schaft 3 und einem Handgriff 4. Das Endoskop 2 ist beispielsweise ein Videoendoskop, ferner beispielsweise ein Stereo-Videoendoskop. An einem distalen Ende des Schafts 3 befindet sich ein optisches System 10.

In Fig. 2 ist schematisch und vereinfacht ein optisches System 10 für ein Stereo-Videoendoskop gezeigt. Das optische System 10 umfasst eine distale optische Baugruppe 12 und eine proximale optische Baugruppe 14. Das optische System 10, also die distale optischen Baugruppe 12 und die proximale optische Baugruppe 14, befindet sich in einem Teil oder Abschnitt des gesamten optischen Systems des Stereo-Videoendoskops, in dem die beiden optischen Kanäle (linker Kanal und rechter Kanal) separat geführt werden.

Die distale optische Baugruppe 12 umfasst eine Anzahl von in Fig. 2 nicht dargestellten optischen Elementen. Aus einem Behandlungs- und/oder Beobachtungsraum an einer distalen Spitze des Schafts 3 einfallende Lichtbündel werden von der distalen optischen Baugruppe 12 empfangen und an die proximale optische Baugruppe 14 weitergeleitet. Die proximale optische Baugruppe 14 umfasst zwei Bildsensoren 40, von denen in Fig. 2 lediglich einer sichtbar ist. Die Bildsensoren 40 sind mit (beispielsweise flexiblen) Leiterplatten 80 zur Weiterleitung von Bildsignalen in Richtung des Handgriffs 4 gekoppelt, beispielsweise auf den betreffenden Leiterplatten 80 montiert.

Die in Fig. 2 gezeigte Ausführungsform des optischen Systems 10 umfasst eine proximale optische Baugruppe 14 mit einer ersten Halterung 20 und einer zweiten Halterung 21, die zur Aufnahme der Bildsensoren 40 ausgebildet sind. Um die proximale optische Baugruppe 14 an der distalen optischen Baugruppe 12 zu fixieren, weisen die erste Halterung 20 und die zweite Halterung 21 jeweils einen ersten Fixierbereich 26 und einen zweiten Fixierbereich 27 auf. Die Fixierbereiche 26, 27 sind zur Aufnahme eines zylinderförmigen Fixierelements 15 ausgebildet.

In der in Fig. 2 gewählten Darstellung ist lediglich der erste Fixierbereich 26 der zweiten Halterung 21 und der zweite Fixierbereich 27 der ersten Halterung 20 sichtbar, die mit einem ersten Fixierelement 15 verlötet sind. Das zweite Fixierelement 16 ist in Fig. 2 verdeckt. Die Fixierelemente 15, 16 werden an der distalen optischen Baugruppe 12 befestigt und jeweils am ersten Fixierbereich 26 einer Halterung 20, 21 und am zweiten Fixierbereich 27 der anderen Halterung 20, 21 mit einer Lötverbindung fixiert. Die Fixierelemente 15, 16 erstrecken sich beispielsweise in eine Richtung parallel zu einer Längsachse 56 des optischen Systems 10. Auch eine Ausrichtung schräg zur Längsachse 56 des optischen Systems 10 kann vorgesehen sein. Auf diese Weise wird eine sehr stabile und platzsparende Anordnung der Halterungen 20, 21 erreicht.

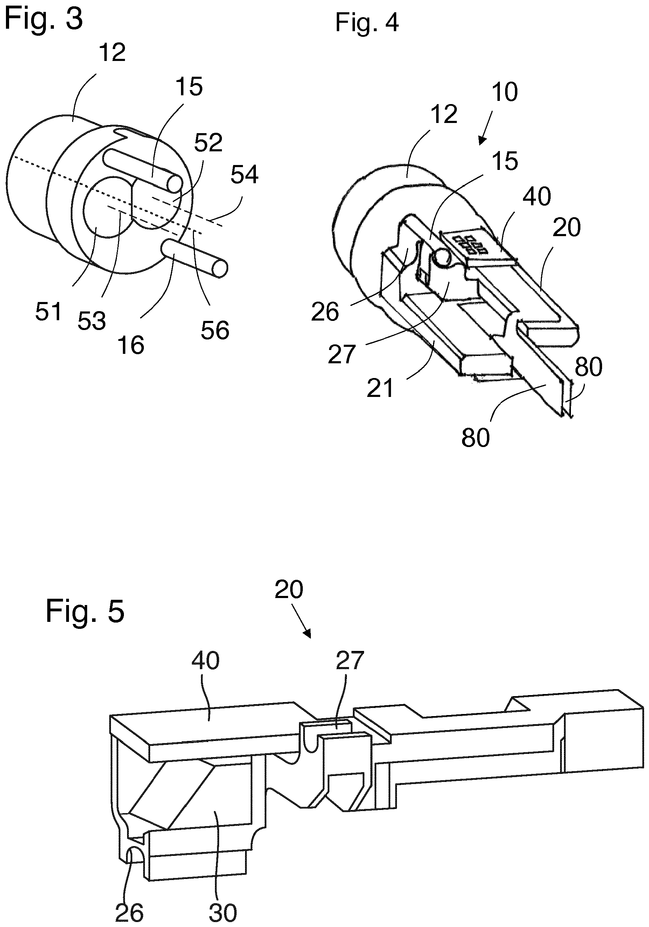

Fig. 3 zeigt eine schematisch vereinfachte Darstellung der distalen optischen Baugruppe 12, in der die beiden Fixierelemente 15, 16 zu erkennen sind. Zudem ist ein erster Linsensystemkanal 51 mit einer ersten optischen Achse 53 und ein zweiter Linsensystemkanal 52 mit einer zweiten optischen Achse 54 dargestellt. Eine Längsachse 56 der optischen Baugruppe 12 erstreckt sich mittig zwischen den beiden optischen Achsen 53, 54. Dabei ist das erste Fixierelement 15 oberhalb und das zweite Fixierelement 16 unterhalb der Linsensystemkanäle 51, 52 angeordnet.

In Fig. 4 ist eine weitere schematisch vereinfachte perspektivische Darstellung des optischen Systems 10 gezeigt. In dieser Darstellung ist zu erkennen, dass jeweils eine Leiterplatte 80 mit einem Bildsensor 40 verbunden ist. Da die Bildsensoren 40 parallel zur Längsachse 56 angeordnet sind, sind in den Halterungen 20, 21 Umlenkprismen angeordnet, die in den Fig. 2 bis 4 verdeckt sind. Die Umlenkprismen lenken die entlang der optischen Achsen 53, 54 einfallenden Lichtbündel in Richtung der Bildsensoren 40 um.

In Fig. 5 ist eine schematische Darstellung einer Halterung 20 gezeigt. Zu erkennen ist die Anordnung des ersten Fixierbereichs 26, des zweiten Fixierbereichs 27 sowie des Bildsensors 40. Zudem ist ein optisches Element 30 in Form eines Umlenkprismas gezeigt, das einfallende Lichtbündel in Richtung des Bildsensors 40 umlenkt.

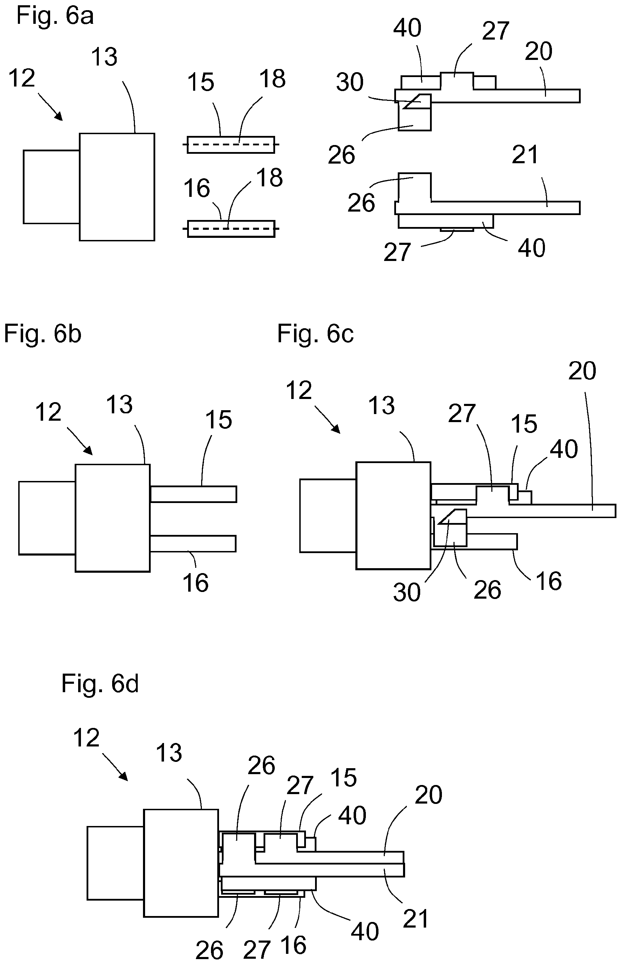

Fig. 6a bis 6d zeigen schematisch ein Verfahren, das nicht unter den Gegenstand der Ansprüche fällt, zum Fixieren einer proximalen optischen Baugruppe an einer distalen optischen Baugruppe am Beispiel des in Fig. 2 bis 4 dargestellten optischen Systems 10, das in Fig. 5a bis 5c in einer vereinfachten Seitenansicht dargestellt ist.

Fig. 6a zeigt die Komponenten, die vor der Durchführung des Verfahrens hergestellt werden. Gezeigt ist die distale optische Baugruppe 12 mit der Fassung 13. In der Fassung 13 ist beispielsweise ein Objektiv angeordnet. Zudem werden die Fixierelemente 15, 16 aus einem Grundmaterial hergestellt und falls erforderlich mit einer Beschichtung versehen, wobei die Beschichtung eine bessere Lötbarkeit als das Grundmaterial aufweist. Die Fixierelemente 15, 16 weisen eine zylinderförmige Form mit einer Zylinderachse 18 auf. Auch die Halterungen 20, 21 werden bereitgestellt, wobei in den Halterungen 20, 21 jeweils ein Bildsensor 40 und ein optisches Element 30, in diesem Fall ein Umlenkprisma, aufgenommen sind. Bei der Halterung 21 ist das optische Element 30 auf Grund des Blickwinkels verdeckt.

Wie in Fig. 6b gezeigt, werden zu Beginn die Fixierelemente 15, 16 an der Fassung 13 fixiert, beispielsweise indem die Fixierelemente 15, 16 in Vertiefungen der Fassung 13 eingeschweißt, eingelötet oder eingeklebt werden.

Im Anschluss wird, wie in Fig. 6c gezeigt, die erste Halterung 20 an den Fixierelementen 15, 16 befestigt. Dazu wird zunächst die Ausrichtung der ersten Halterung 20 justiert. Beispielsweise wird die optische Weglänge von dem Objektiv in der Fassung 13 über das optische Element 30 zu dem auf der ersten Halterung 20 angeordneten Bildsensor 40 eingestellt. Anschließend wird der erste Fixierbereich 26 der ersten Halterung 20 an dem zweiten Fixierelement 16 und der zweite Fixierbereich 27 der ersten Halterung 20 an dem ersten Fixierelement 15 mittels einer Lötverbindung fixiert.

Zuletzt wird die zweite Halterung an den Fixierelementen 15, 16 befestigt, wie in Fig. 6d gezeigt. Dabei wird wie bei der ersten Halterung 20 zunächst die Ausrichtung der zweiten Halterung 21 justiert und anschließend der erste Fixierbereich 26 der zweiten Halterung 21 an dem ersten Fixierelement 15 und der zweite Fixierbereich 27 der zweiten Halterung 21 an dem zweiten Fixierelement 16 mittels einer Lötverbindung fixiert. Durch die gekreuzte Anordnung der Fixierbereiche 26, 27 jedes der Halterungen 20, 21 wird eine hohe Stabilität und große Verwindungssteifigkeit erreicht.

### Bezugszeichenliste

- 2: Endoskop
- 3: Schaft
- 4: Handgriff
- 10: optisches System
- 12: distale optische Baugruppe
- 13: Fassung
- 14: proximale optische Baugruppe
- 15: erstes Fixierelement
- 16: zweites Fixierelement
- 18: Zylinderachse
- 20: erste Halterung
- 21: zweite Halterung
- 26: erster Fixierbereich
- 27: zweiter Fixierbereich
- 30: optisches Element
- 40: Bildsensor
- 51: erster Linsensystemkanal
- 52: zweiter Linsensystemkanal
- 53: erste optische Achse
- 54: zweite optische Achse
- 56: Längsachse
- 80: Leiterplatte

## Patentansprüche

1. Optisches System (10) für ein Endoskop (2), umfassend eine distale optische Baugruppe (12) und eine proximale optische Baugruppe (14), wobei das optische System (10) wenigstens ein stabförmiges Fixierelement (15, 16) mit einer längserstreckten Form aufweist, wobei mittels des stabförmigen Fixierelements (15, 16) die proximale optische Baugruppe (14) an der distalen optischen Baugruppe (12) befestigt ist und die proximale optische Baugruppe (14) eine erste Halterung (20) und eine zweite Halterung (21) umfasst, die jeweils zur Aufnahme eines Bildsensors (40) eingerichtet sind, wobei ferner die erste Halterung (20) und die zweite Halterung (21) aneinander fixiert sind,
**dadurch gekennzeichnet, dass**
das wenigstens eine stabförmige Fixierelement (15, 16) hohlraumfrei ist und die erste Halterung (20) gegenüber der zweiten Halterung (21) achsensymmetrisch in Bezug auf eine Längsachse (56) des optischen Systems (10) angeordnet ist.

2. Optisches System (10) nach Anspruch 1, **dadurch gekennzeichnet, dass** sich das stabförmige Fixierelement (15, 16) in eine Richtung parallel zu der Längsachse (56) des optischen Systems (10) erstreckt.

3. Optisches System (10) nach Anspruch 2, **dadurch gekennzeichnet, dass** das stabförmige Fixierelement (15, 16) eine zylinderförmige Form aufweist, wobei sich eine Zylinderachse (18) des stabförmigen Fixierelements (15, 16) parallel zu der Längsachse (56) des optischen Systems (10) erstreckt.

4. Optisches System (10) nach einem der Ansprüche 1 bis 3, **dadurch gekennzeichnet, dass** das stabförmige Fixierelement (15, 16) einstückig mit der distalen optischen Baugruppe (12) hergestellt ist.

5. Optisches System (10) nach einem der Ansprüche 1 bis 3, **dadurch gekennzeichnet, dass** das stabförmige Fixierelement (15, 16) als separates Element hergestellt und in einer Fassung (13) der distalen optischen Baugruppe (12) fixiert, insbesondere eingeschweißt, ist.

6. Optisches System (10) nach Anspruch 5, **dadurch gekennzeichnet, dass** das stabförmige Fixierelement (15, 16) aus einem Grundmaterial hergestellt und mit einer Beschichtung versehen ist, wobei die Beschichtung eine bessere Lötbarkeit als das Grundmaterial aufweist.

7. Optisches System (10) nach Anspruch 6, **dadurch gekennzeichnet, dass** das stabförmige Fixierelement (15, 16) aus dem Grundmaterial und die Fassung (13) der distalen optischen Baugruppe (12) aus einem Fassungsmaterial hergestellt ist, wobei sich das Grundmaterial von dem Fassungsmaterial unterscheidet.

8. Optisches System (10) nach einem der Ansprüche 1 bis 7, **dadurch gekennzeichnet, dass** das stabförmige Fixierelement (15, 16) mittels einer Lötverbindung an der proximalen optischen Baugruppe (14) fixiert ist.

9. Optisches System (10) nach Anspruch 1, **dadurch gekennzeichnet, dass** das wenigstens eine stabförmige Fixierelement (15, 16) ein erstes stabförmiges Fixierelement (15) und ein zweites stabförmiges Fixierelement (16) umfasst, wobei das erste stabförmige Fixierelement (15) die erste Halterung (20) und das zweite stabförmige Fixierelement (16) die zweite Halterung (21) an der distalen optischen Baugruppe (12) fixiert.

10. Optisches System (10) nach Anspruch 1, **dadurch gekennzeichnet, dass** das wenigstens eine stabförmige Fixierelement (15, 16) ein erstes stabförmiges Fixierelement (15) und ein zweites stabförmiges Fixierelement (16) umfasst, wobei die erste und zweite Halterung (20, 21) jeweils einen ersten Fixierbereich (26) und einen zweiten Fixierbereich (27) aufweist, und wobei das erste stabförmige Fixierelement (15) die distale optische Baugruppe (12) an dem ersten Fixierbereich (26) der zweiten Halterung (21) und dem zweiten Fixierbereich (27) der ersten Halterung (20) fixiert, und wobei das zweite stabförmige Fixierelement (16) die distale optische Baugruppe (12) an dem ersten Fixierbereich (26) der ersten Halterung (20) und dem zweiten Fixierbereich (27) der zweiten Halterung (21) fixiert.

11. Optisches System (10) nach Anspruch 10, **dadurch gekennzeichnet, dass** das optische System (10) einen ersten Linsensystemkanal (51) mit einer ersten optischen Achse (53) und einen zweiten Linsensystemkanal (52) mit einer zweiten optischen Achse (54) umfasst, wobei die erste optische Achse (53) parallel zur zweiten optischen Achse (54) und zur Längsachse (56) des optischen Systems (10) verläuft, wobei das erste stabförmige Fixierelement (15) oberhalb des ersten Linsensystemkanals (51) und das zweite stabförmige Fixierelement (16) unterhalb des zweiten Linsensystemkanals (52) angeordnet ist.

12. Endoskop (2), insbesondere Stereo-Videoendoskop, umfassend ein optisches System (10) nach einem der Ansprüche 1 bis 11.

## Claims

1. An optical system (10) for an endoscope (2), comprising a distal optical assembly (12) and a proximal optical assembly (14), wherein the optical system (10) has at least one bar-shaped fixing element (15, 16) having an elongate form, wherein the proximal optical assembly (14) is fastened to the distal optical assembly (12) by means of the bar-shaped fixing element (15, 16) and the proximal optical assembly (14) comprises a first holder (20) and a second holder (21) which are each designed to receive an image sensor (40), wherein the first holder (20) and the second holder (21) are fixed to one another, **characterized in that** the at least one bar-shaped fixing element (15, 16) is free from hollow spaces and the first holder (20) is arranged axisymmetrically with respect to the second holder (21) in relation to a longitudinal axis (56) of the optical system (10).

2. The optical system (10) according to Claim 1, **characterized in that** the bar-shaped fixing element (15, 16) extends in a direction parallel to the longitudinal axis (56) of the optical system (10).

3. The optical system (10) according to Claim 2, **characterized in that** the bar-shaped fixing element (15, 16) has a cylindrical form, wherein a cylinder axis (18) of the bar-shaped fixing element (15, 16) extends parallel to the longitudinal axis (56) of the optical system (10).

4. The optical system (10) according to any one of Claims 1 to 3, **characterized in that** the bar-shaped fixing element (15, 16) is manufactured integrally with the distal optical assembly (12).

5. The optical system (10) according to any one of Claims 1 to 3, **characterized in that** the bar-shaped fixing element (15, 16) is manufactured as a separate element and is fixed, in particular welded, in a mount (13) of the distal optical assembly (12).

6. The optical system (10) according to Claim 5, **characterized in that** the bar-shaped fixing element (15, 16) is manufactured from a base material and provided with a coating, wherein the coating has better solderability than the base material.

7. The optical system (10) according to Claim 6, **characterized in that** the bar-shaped fixing element (15, 16) is manufactured from the base material and the mount (13) of the distal optical assembly (12) is manufactured from a mount material, wherein the base material differs from the mount material.

8. The optical system (10) according to any one of Claims 1 to 7, **characterized in that** the bar-shaped fixing element (15, 16) is fixed to the proximal optical assembly (14) by means of a soldered connection.

9. The optical system (10) according to Claim 1, **characterized in that** the at least one bar-shaped fixing element (15, 16) comprises a first bar-shaped fixing element (15) and a second bar-shaped fixing element (16), wherein the first bar-shaped fixing element (15) fixes the first holder (20) and the second bar-shaped fixing element (16) fixes the second holder (21) to the distal optical assembly (12).

10. The optical system (10) according to Claim 1, **characterized in that** the at least one bar-shaped fixing element (15, 16) comprises a first bar-shaped fixing element (15) and a second bar-shaped fixing element (16), wherein the first and the second holder (20, 21) each has a first fixing region (26) and a second fixing region (27), and wherein the first bar-shaped fixing element (15) fixes the distal optical assembly (12) to the first fixing region (26) of the second holder (21) and to the second fixing region (27) of the first holder (20), and wherein the second bar-shaped fixing element (16) fixes the distal optical assembly (12) to the first fixing region (26) of the first holder (20) and to the second fixing region (27) of the second holder (21).

11. The optical system (10) according to any one of Claims 1 to 10, **characterized in that** the optical system (10) comprises a first lens system channel (51) having a first optical axis (53) and a second lens system channel (52) having a second optical axis (54), wherein the first optical axis (53) runs parallel to the second optical axis (54) and to the longitudinal axis (56) of the optical system (10), wherein the first bar-shaped fixing element (15) is arranged above the first lens system channel (51) and the second bar-shaped fixing element (16) is arranged below the second lens system channel (52).

12. An endoscope (2), in particular a stereo-video endoscope, comprising an optical system (10) according to any one of Claims 1 to 11.

## Revendications

1. Système optique (10) pour un endoscope (2), comprenant un ensemble optique distal (12) et un ensemble optique proximal (14), le système optique (10) présentant au moins un élément de fixation (15, 16) en forme de tige ayant une forme allongée, l'ensemble optique proximal (14) étant fixé à l'ensemble optique distal (12) au moyen de l'élément de fixation (15, 16) en forme de tige, et l'ensemble optique proximal (14) comprenant un premier support (20) et un deuxième support (21), qui sont respectivement conçus pour recevoir un capteur d'image (40),
le premier support (20) et le deuxième support (21) étant en outre fixés l'un à l'autre,
**caractérisé en ce que** ledit au moins un élément de fixation (15, 16) en forme de tige est exempt d'espace creux et le premier support (20) est disposé de manière axisymétrique par rapport au deuxième support (21) par rapport à un axe longitudinal (56) du système optique (10).

2. Système optique (10) selon la revendication 1, **caractérisé en ce que** l'élément de fixation (15, 16) en forme de tige s'étend dans une direction parallèle à l'axe longitudinal (56) du système optique (10).

3. Système optique (10) selon la revendication 2, **caractérisé en ce que** l'élément de fixation (15, 16) en forme de tige présente une forme cylindrique, un axe cylindrique (18) de l'élément de fixation (15, 16) en forme de tige s'étendant parallèlement à l'axe longitudinal (56) du système optique (10).

4. Système optique (10) selon l'une quelconque des revendications 1 à 3, **caractérisé en ce que** l'élément de fixation (15, 16) en forme de tige est fabriqué d'un seul tenant avec l'ensemble optique distal (12).

5. Système optique (10) selon l'une des revendications 1 à 3, **caractérisé en ce que** l'élément de fixation (15, 16) en forme de tige est fabriqué en tant qu'élément séparé et est fixé, en particulier soudé, dans une monture (13) de l'ensemble optique distal (12).

6. Système optique (10) selon la revendication 5, **caractérisé en ce que** l'élément de fixation (15, 16) en forme de tige est fabriqué à partir d'un matériau de base et est pourvu d'un revêtement, le revêtement présentant une meilleure soudabilité que le matériau de base.

7. Système optique (10) selon la revendication 6, **caractérisé en ce que** l'élément de fixation (15, 16) en forme de tige est fabriqué à partir du matériau de base et la monture (13) de l'ensemble optique distal (12) est fabriquée à partir d'un matériau de monture, le matériau de base étant différent du matériau de monture.

8. Système optique (10) selon l'une quelconque des revendications 1 à 7, **caractérisé en ce que** l'élément de fixation (15, 16) en forme de tige est fixé à l'ensemble optique proximal (14) au moyen d'une jonction par soudure.

9. Système optique (10) selon la revendication 1, **caractérisé en ce que** ledit au moins un élément de fixation (15, 16) en forme de tige comprend un premier élément de fixation (15) en forme de tige et un deuxième élément de fixation (16) en forme de tige, le premier élément de fixation (15) en forme de tige fixant le premier support (20) et le deuxième élément de fixation (16) en forme de tige fixant le deuxième support (21) sur l'ensemble optique distal (12).

10. Système optique (10) selon la revendication 1, **caractérisé en ce que** ledit au moins un élément de fixation (15, 16) en forme de tige comprend un premier élément de fixation (15) en forme de tige et un deuxième élément de fixation (16) en forme de tige, lesdits premier et deuxième supports (20, 21) ayant chacun une première zone de fixation (26) et une deuxième zone de fixation (27), et le premier élément de fixation (15) en forme de tige fixe l'ensemble optique distal (12) à la première zone de fixation (26) du deuxième support (21) et à la deuxième zone de fixation (27) du premier support (20), et le deuxième élément de fixation (16) en forme de tige fixe l'ensemble optique distal (12) à la première zone de fixation (26) du premier support (20) et à la deuxième zone de fixation (27) du deuxième support (21).

11. Système optique (10) selon la revendication 10, **caractérisé en ce que** le système optique (10) comprend un premier canal (51) de système de lentilles ayant un premier axe optique (53) et un deuxième canal (52) de système de lentilles ayant un deuxième axe optique (54), le premier axe optique (53) étant parallèle au deuxième axe optique (54) et à l'axe longitudinal (56) du système optique (10), le premier élément de fixation (15) en forme de tige étant agencé au-dessus du premier canal (51) de système de lentilles et le deuxième élément de fixation (16) en forme de tige étant agencé au-dessous du deuxième canal (52) de système de lentilles.

12. Endoscope (2), en particulier endoscope vidéo stéréoscopique, comprenant un système optique (10) selon l'une des revendications 1 à 11.
